# EUROPEAN PATENT SPECIFICATION

(11) **EP 3 545 528 B1**
(45) Date of publication and mention of the grant of the patent: **28.10.2026**
(21) Application number: 17822091.9
(22) Date of filing: 28.11.2017
(51) Int. Cl.: G16H 50/20, A61B 5/055

(54) **SYSTEMS AND METHODS FOR IDENTIFYING A NEUROPHYSIOLOGICAL BIOTYPE OF DEPRESSION IN THE BRAIN OF A PATIENT**
SYSTEME UND VERFAHREN ZUR IDENTIFIZIERUNG EINES NEUROPHYSIOLOGISCHEN BIOTYPS VON DEPRESSION IM GEHIRN EINES PATIENTEN
SYSTÈMES ET PROCÉDÉS D'IDENTIFICATION D'UN BIOTYPE NEUROPHYSIOLOGIQUE DE DÉPRESSION DANS LE CERVEAU D'UN PATIENT

(30) Priority: 28.11.2016 US 201662426835 P
(43) Date of publication of application: 02.10.2019
(73) Proprietor: Cornell University, Ithaca, NY 14850 (US)
(72) Inventor: LISTON, Conor, Ithaca, New York 14850 (US)
(74) Representative: Betten & Resch
(86) International application number: PCT/US2017/063425
(87) International publication number: WO 2018/098467

(56) References cited:
- US-A1- 2016 019 693
- LEANNE M WILLIAMS: "Defining biotypes for depression and anxiety based on large-scale circuit dysfunction: a theoretical review of the evidence and future directions for clinical translation", DEPRESSION AND ANXIETY, vol. 34, no. 1, 21 September 2016 (2016-09-21), US, pages 9 - 24, XP055678010, ISSN: 1091-4269, DOI: 10.1002/da.22556
- LONGLONG CAO ET AL: "Aberrant functional connectivity for diagnosis of major depressive disorder: A discriminant analysis : Potential new biomarkers for MDD", PSYCHIATRY AND CLINICAL NEUROSCIENCES, vol. 68, no. 2, 31 October 2013 (2013-10-31), AU, pages 110 - 119, XP055454781, ISSN: 1323-1316, DOI: 10.1111/pcn.12106
- RAJAMANNAR RAMASUBBU ET AL: "Accuracy of automated classification of major depressive disorder as a function of symptom severity", NEUROIMAGE: CLINICAL, vol. 12, 1 February 2016 (2016-02-01), pages 320 - 331, XP055453982, ISSN: 2213-1582, DOI: 10.1016/j.nicl.2016.07.012
- WEI MAOBIN ET AL: "Identifying major depressive disorder using Hurst exponent of resting-state brain networks", PSYCHIATRY RESEARCH: NEUROIMAGING, vol. 214, no. 3, 2013, pages 306 - 312, XP028796214, ISSN: 0925-4927, Retrieved from the Internet <URL:Identifying major depressive disorder using Hurst exponent of resting-state brain networks> DOI: 10.1016/J.PSCYCHRESNS.2013.09.008
- ANTON LORD ET AL: "Changes in Community Structure of Resting State Functional Connectivity in Unipolar Depression", PLOS ONE, vol. 7, no. 8, 20 August 2012 (2012-08-20), pages e41282, XP055454187, DOI: 10.1371/journal.pone.0041282
- NATHAN BAKKER: "Resting-State Functional Connectivity Predicts Individual Treatment Outcomes of Repetitive Transcranial Magnetic Stimulation for Major Depressive Disorder", 1 January 2014 (2014-01-01), XP055454305, ISBN: 978-1-339-52188-6, Retrieved from the Internet <URL:https://search.proquest.com/docview/1771278068?accountid=29404>
- DOMINIK GROTEGERD ET AL: "Discriminating unipolar and bipolar depression by means of fMRI and pattern classification: a pilot study", EUROPEAN ARCHIVES OF PSYCHIATRY AND CLINICAL NEUROSCIENCE., vol. 263, no. 2, 26 May 2012 (2012-05-26), DE, pages 119 - 131, XP055454016, ISSN: 0940-1334, DOI: 10.1007/s00406-012-0329-4
- CONOR LISTON ET AL: "Default Mode Network Mechanisms of Transcranial Magnetic Stimulation in Depression", BIOLOGICAL PSYCHIATRY, vol. 76, no. 7, 1 October 2014 (2014-10-01), NEW YORK, NY; US, pages 517 - 526, XP055454007, ISSN: 0006-3223, DOI: 10.1016/j.biopsych.2014.01.023
- TELMA ALVES VELOSO: "Exploration and application of machine learning algorithms to functional connectivity data", 1 January 2014 (2014-01-01), XP055454200, Retrieved from the Internet <URL:https://repositorium.sdum.uminho.pt/bitstream/1822/34155/1/Telma%20Alves%20Veloso.pdf> [retrieved on 20180226]
- BENNY LIBERG ET AL: "Functional and Structural Alterations in the Cingulate Motor Area Relate to Decreased Fronto-Striatal Coupling in Major Depressive Disorder with Psychomotor Disturbances", FRONTIERS IN PSYCHIATRY, vol. 5, 4 December 2014 (2014-12-04), XP055454013, DOI: 10.3389/fpsyt.2014.00176
- OATHES DESMOND J ET AL: "Neurobiological Signatures of Anxiety and Depression in Resting-State Functional Magnetic Resonance Imaging", BIOLOGICAL PSYCHIATRY, ELSEVIER SCIENCE, NEW YORK, NY; US, vol. 77, no. 4, 15 August 2014 (2014-08-15), pages 385 - 393, XP029226222, ISSN: 0006-3223, DOI: 10.1016/J.BIOPSYCH.2014.08.006

## Description

### RELATED APPLICATIONS

The present application claims priority to, and the benefit of, U.S. Provisional Patent Application No. 62/426,835, entitled "NEUROIMAGING BIOMARKERS FOR DIAGNOSING DEPRESSION SUBTYPES AND PREDICTING AND QUANTIFYING TREATMENT RESPONSE" and filed on November 28, 2016.

### BACKGROUND

Depression diagnosis continues to be a challenge for physicians and psychiatrists. Experts have concerns over the traditional subjective clinical symptom approach to depression diagnosis, especially for diagnosing depression biotypes. More objective measures are needed to detect a subject's depression and predict their treatment response.

Longlong Cao et al.: "Aberrant functional connectivity for diagnosis of major depressive disorder: A discriminant analysis", PSYCHIATRY AND CLINICAL NEUROSCIENCES, vol.68, no. 2, 31 October 2013, pages 110-119 discloses an examination of the efficiency of discriminant analysis of major depressive disorder by individualized computer-assisted diagnosis.

Leanne M Williams: "Defining biotypes for depression and anxiety based on large-scale circuit dysfunction: A theoretical review of the evidence and future directions for clinical translation", DEPRESSION AND ANXIETY, vol. 34, no. 1, 21 September 2016, pages 9-24 suggests a conceptual framework is for considering neural circuit-defined biotypes defined by profiles of extent of dysfunction on each large-scale circuit. The clinical implications of a biotype approach for guiding classification and treatment of depression and anxiety are considered.

### SUMMARY

According to one aspect, the disclosure relates to a method for identifying a neurophysiological biotype of depression in the brain of a patient as recited in claim 1. Further embodiments of the disclosure are recited in dependent claims 2-9

The biotype classifier includes a plurality of classifiers. The plurality of classifiers include a first set of classifiers, where each classifier is configured to generate a biotype depression likelihood score for a different corresponding depression biotype. The depression biotype is identified by selecting the depression biotype associated with the classifier that generates the highest biotype depression likelihood score based on the extracted brain region functional connectivity information. In some implementations, the extracted brain region functional connectivity information includes functional connectivity levels between a plurality of the following patient brain regions: the dorsomedial prefrontal cortex, middle temporal gyrus, parahippocampal cortex, dorsolateral prefrontal cortex, anterior prefrontal cortex, posterior parietal cortex, anterior cingulate cortex, insular cortex, ventrolateral prefrontal cortex, precuneus, orbitofrontal cortex, ventral striatum thalamus, raphe nucleus (midbrain), primary cortex, somatosensory cortex, and visual cortex.

In some implementations, the method includes processing the extracted brain functional connectivity information by an additional classifier included in a second set of classifiers configured for determining if the patient suffers from depression, wherein the additional classifier is associated with the depression biotype identified for the patient.

In some implementations, determining if the patient suffers from a first biotype of depression or if the patient has no known depression diagnosis, includes processing the extracted brain region functional connectivity information between a plurality of the following patient brain regions: the posterior parietal cortex, precuneus, middle temporal gyrus, parahippocampal cortex, dorsolateral prefrontal cortex, ventrolateral prefrontal cortex, insular cortex, temporal pol, superior temporal gyrus, ventromedial prefrontal cortex, ventral hippocampus, amygdala, ventral caudate nucleus, ventral striatum, thalamus, primary somatosensory cortex, primary motor cortex, visual cortex.

In some implementations, determining if the patient suffers from a second biotype or if the patient has no known depression diagnosis, includes processing the extracted brain region functional connectivity information between a plurality of the following patient brain regions: the dorsomedial prefrontal cortex, ventromedial prefrontal cortex, orbitofrontal cortex, posterior cingulate cortex, fusiform gyrus, middle temporal gyrus, parahippocampal cortex, anterior cingulate cortex, ventrolateral prefrontal cortex, dorsolateral prefrontal cortex, insular cortex, posterior parietal cortex, thalamus, primary somatosensory cortex, primary motor cortex, and visual cortex.

In some implementations, determining if the patient suffers from a third biotype of depression or if the patient has no known depression diagnosis, includes processing the extracted brain region functional connectivity information between a plurality of the following patient brain regions: the ventromedial prefrontal cortex, posterior parietal cortex, middle temporal gyrus, parahippocampal cortex, ventrolateral prefrontal cortex, anterior prefrontal cortex, dorsolateral prefrontal cortex, posterior parietal cortex, temporal pole, superior temporal gyrus, orbitofrontal cortex, ventral hippocampus, amygdala, subgenual anterior cingulate cortex, ventral caudate nucleus, ventral striatum, thalamus, premotor cortex, supplementary motor area, insular cortex, anterior cingulate cortex, and fusiform gyrus.

In some implementations, determining if the patient suffers from a fourth biotype of depression or if the patient has no known depression diagnosis, processing the extracted brain region functional connectivity information between a plurality of the following patient brain regions: the fusiform gyrus, parahippocampal cortex, ventrolateral prefrontal cortex, posterior parietal cortex, anterior cingulate cortex, middle cingulate cortex, insular cortex, temporal pole, superior temporal gyrus, middle temporal gyrus, orbitofrontal cortex, ventral hippocampus, subgenual anterior cingulate cortex, ventral striatum, thalamus, primary somatosensory cortex, supplementary motor area, insular cortex, postcentral gyrus, and lingual gyrus.

In some implementations, the biotype classifier may include a linear support vector machine classifier configured to output the likelihood of an associated depression biotype.

### BRIEF DESCRIPTION OF THE DRAWINGS

The skilled artisan will understand that the figures, described herein, are for illustration purposes only. It is to be understood that in some instances various aspects of the described implementations may be shown exaggerated or enlarged to facilitate an understanding of the described implementations. In the drawings, like reference characters generally refer to like features, functionally similar and/or structurally similar elements throughout the various drawings. The drawings are not necessarily to scale, emphasis instead being placed upon illustrating the principles of the teachings. The drawings are not intended to limit the scope of the present teachings in any way. The system and method may be better understood from the following illustrative description with reference to the following drawings in which:
Figure 1 illustrates a first schematic diagram of an example diagnostic system for classifying a subject into a depression biotype and predicting a treatment prognosis.
Figure 2 illustrates a second schematic diagram of an example diagnostic system for classifying a subject into a depression biotype and predicting a treatment prognosis.
Figure 3 illustrates a flow chart of an example method for identifying depression biotypes in a subject.
Figure 4 illustrates a flow chart of an example method for identifying a depression biotype and predicting a treatment prognosis.
Figure 5 illustrates a flow chart of an example method for identifying a subject's response to a depression treatment option.
Figure 6 illustrates a flow chart of an example method for predicting a subject's depression severity and tracking it over time.
Figure 7 illustrates a block diagram of an example computing system.

### DETAILED DESCRIPTION OF THE DRAWINGS

The various concepts introduced above and discussed in greater detail below may be implemented in various ways, as the described concepts are not limited to any particular manner of implementation. Examples of specific implementations are provided for illustrative purposes.

Depression is a heterogeneous clinical syndrome linked to dysfunction and abnormal connectivity in frontostiatal and limbic networks of the brain. Depression is typically diagnosed when a patient suffers from at least five of nine symptoms - allowing for several hundred unique combinations of changes in mood, appetite, sleep, energy, cognition, and motor activity. Diagnostic heterogeneity has emerged as a major obstacle to understanding the pathophysiology of major mental illnesses and depression. Although major depressive disorders, especially highly recurrent depression, is up to 45% heritable, identifying genetic risk factors has proven challenging, even in extremely large genome-wide association studies. Likewise, efforts to develop new treatments have slowed, due in part to a lack of physiological targets for assessing treatment efficacy and selecting individual patients who are most likely to benefit. All of these challenges have been attributed in part to the fact that our current diagnostic system assigns a single label to a syndrome that is not unitary and may be caused by distinct pathological processes requiring different treatments.

The present system and methods described herein diagnose and predict treatment responses for patients suffering with depression by classifying the patient into a depression biotype based on unique patterns of abnormal connectivity in brain networks, including resting state brain networks. Functional connectivity may reflect the relationship between neuronal activation patterns in two or more regions of the brain over time evaluated by a functional magnetic resonance imaging or functional MRI (fMRI).

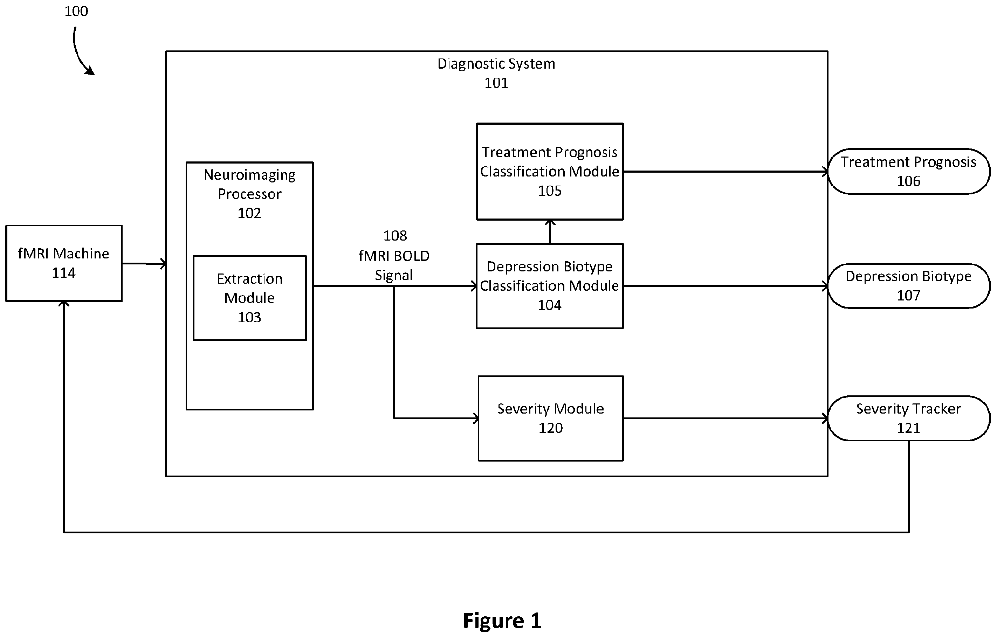

Figure 1 illustrates an example block diagram of the system 100 for diagnosing depression biotypes in the brain and predicting treatment responses. The system 100 includes a diagnostic system 101, which includes a neuroimaging processor 102, depression biotype classification module 104, a treatment prognosis classification module 105, and a severity module 120. The diagnostic system 101 receives fMRI data from a subject undergoing an fMRI in an fMRI machine 114.

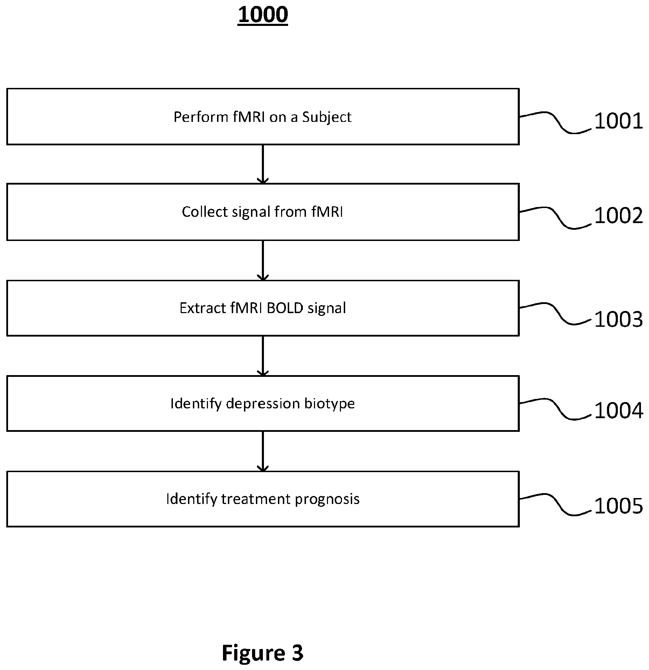

The system 100 includes a diagnostic system 101. The diagnostic system 101 collects fMRI data from an fMRI machine 114. The fMRI machine 114 uses magnetic resonance imaging to map neural networks of a subject's brain and records in vivo a whole brain scan of an individual. The neuroimaging processor 102 processes fMRI data. In some implementations, the neuroimaging processor will process the fMRI data to control for motion, age, and scanner related effects, or any combination thereof. The neuroimaging processor 102 includes an extraction module 103. The extraction module 103 extracts the fMRI BOLD signal 108 from the fMRI data. The fMRI BOLD signal includes brain region functional connectivity information. Details of a method for extracting the fMRI BOLD signal are described further in relation to Figure 3.

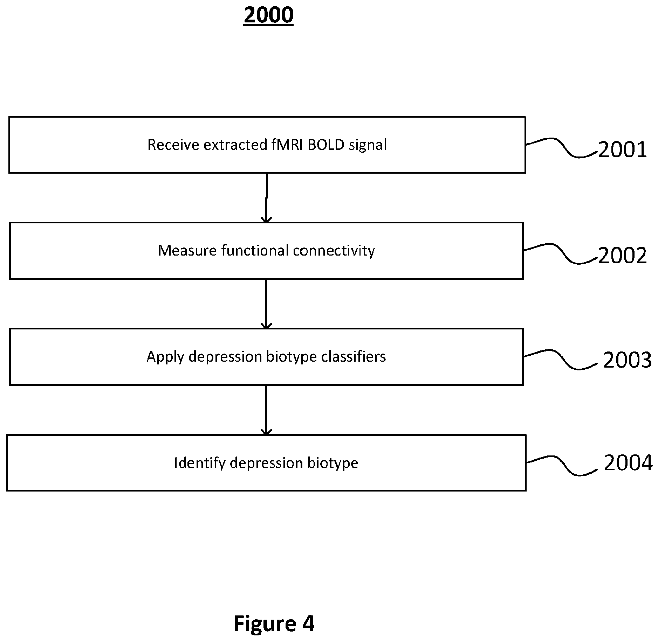

The diagnostic system 101 also includes a depression biotype classification module 104. As mentioned above, the extraction module 103 extracts the fMRI BOLD signal 108 from the fMRI data. The depression biotype classification module 104 receives the fMRI BOLD signal 108 from the neuroimaging processor 102 and extraction module 103. The depression biotype classification module 104 then identifies a depression biotype 107 of the subject. The identified depression biotype includes one of at least four depression biotypes, Biotype 1, Biotype 2, Biotype 3, and Biotype 4. A depression biotype is identified based on patterns of abnormal connectivity in resting state brain networks. Details of a method for identifying depression biotypes and details on the alterations in functional connectivity between different brain regions for identifying depression biotypes are described further in relation to Figure 4.

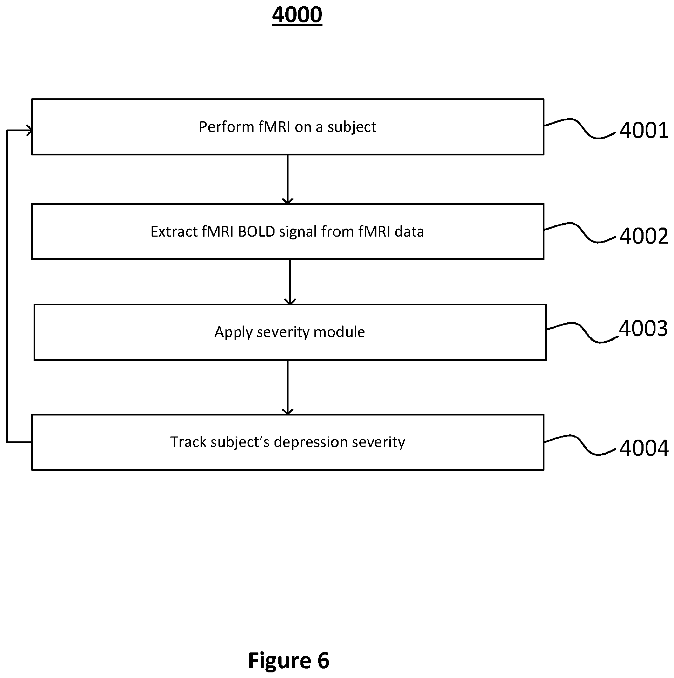

The diagnostic system 110 also includes a severity module 120. The severity module 120 receives the fMRI BOLD signal from the neuroimaging processor 102 and the extraction module 103. The severity module 120 predicts depression severity based on the pairwise fMRI functional connectivity measures. The severity module 120 outputs a predicted severity score to the severity tracker 121. The severity tracker 121 monitors the subject's predicted depression severity over time based on multiple fMRI scans. Details of a method for predicting a subject's depression severity are described further in relation to Figure 6.

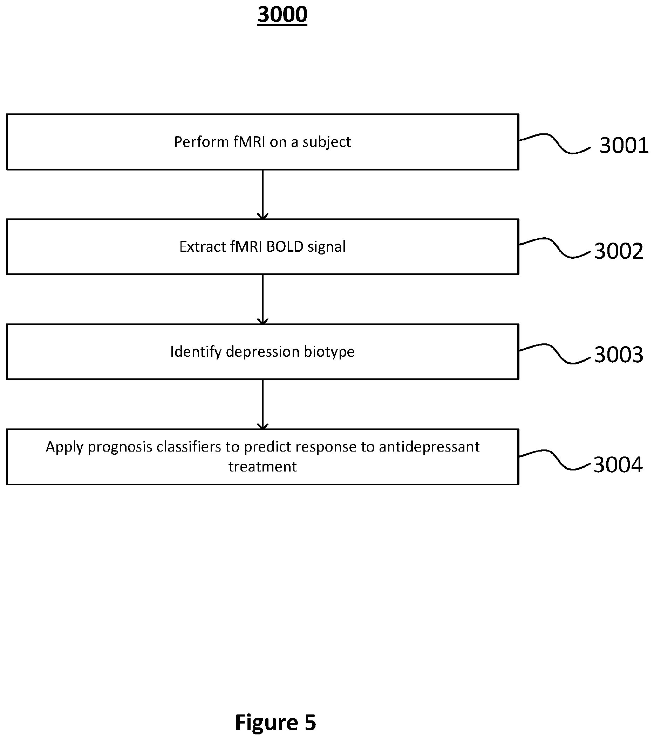

The diagnostic system 101 also includes a treatment prognosis classification module 105. The treatment prognosis classification module 105 receives the identified depression biotype from the depression biotype classification module 104 and predicts a treatment response based on the subject's depression biotype. The treatment prognosis classification module 105 outputs a treatment prognosis 106. The treatment prognosis 106 informs the subject or their clinician of the likelihood of success of an identified depressive treatment based on their diagnosed depression biotype. Subjects may be differentially responsive to anti-depressant treatment based on their depression biotypes. Some depression biotypes may exhibit very little treatment response to a particular treatment, while other biotypes may exhibit significant treatment response. In some implementations, the treatment may be repetitive transcranial magnetic stimulation (rTMS) targeting the dorsomedial prefrontal cortex for depression. rTMS is a non-invasive neurostimulation treatment for medication resistant depression. Details of a method for identifying treatment response are described further in relation to Figure 5.

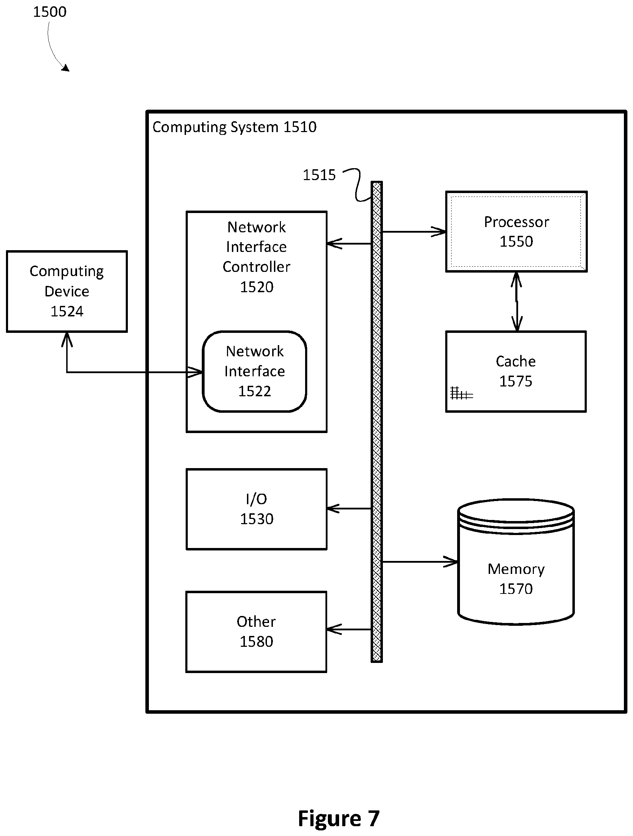

The analysis of the subject's fMRI data may be provided by a computer and outputted by the computer. Details describing the methods of the computer system are described further in Figure 7 below.

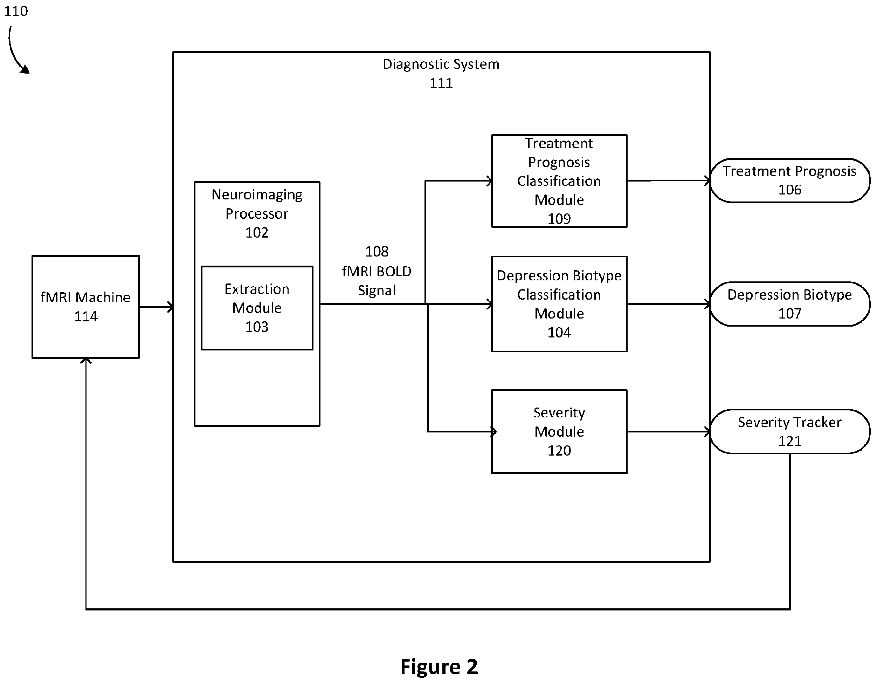

Figure 2 illustrates an example block diagram of a system 110 for identifying depression biotypes in the brain and predicting treatment responses. The system 110 includes a diagnostic system 111. The diagnostic system 111 includes a neuroimaging processor 102, a depression biotype classification module 104, a severity module 120, and a treatment prognosis classification module 109. The diagnostic system receives fMRI data from a subject undergoing an fMRI from an fMRI machine 114.

The system 110 includes a diagnostic system 111. The diagnostic system 111 includes the neuroimaging processor 102. The diagnostic system 111 collects fMRI data from an fMRI machine 114. The neuroimaging processor 102 processes the fMRI data. As mentioned above, in some implementations, the neuroimaging processor will process the data to control for age, motion, and scanner related effects in order to control for artifacts. The neuroimaging processor 102 includes an extraction module 103. The extraction module 103 extracts the fMRI BOLD signal 108 from the fMRI data. Details of a method for extracting the fMRI BOLD signal are described further in relation to Figure 3.

The diagnostic system 111 also includes a treatment prognosis classification module 109 and a depression biotype classification module 104. The neuroimaging processor 102 outputs the fMRI BOLD signal 108 to the depression biotype classification module 104. The depression biotype classification module 104 outputs the identified depression biotype 107. The depression biotype 107 represents an identified depression biotype determined based on the abnormal functional connectivity in the subject's brain. The depression biotype can be one of a plurality of depression biotypes. Details of a method for identifying depression biotypes and details on the alterations in functional connectivity between different brain regions for identifying depression biotypes are described further in relation to Figure 4.

The neuroimaging processor 102 also outputs the fMRI BOLD signal 108 to the treatment prognosis classification module 109. The treatment prognosis classifier processes the fMRI BOLD signal 108 and outputs a treatment prognosis 106. The treatment prognosis predicts the anti-depressant treatment response, of the subject, based on their unique fMRI BOLD data. In some implementations, the treatment may be a non-invasive neurostimulation treatment for medication resistant depression, such as rTMS. A more detailed discussion of methods for classifying a subject's response to a depression treatment option as carried out by the treatment prognosis classifier are described in relation to Figure 5.

The diagnostic system 110 also includes a severity module 120. The severity module 120 receives the fMRI BOLD signal 108 from the neuroimaging processor 102 and the extraction module 103. The severity module 120 outputs a predicted depression severity score, based on the pairwise fMRI functional connectivity measures between different brain regions, to the severity tracker 121. The severity tracker 121 monitors the subject's predicted depression severity over time based on multiple fMRI scans. Details of a method for classifying a subject's depression severity are described further in relation to Figure 6.

The analysis of the subject's fMRI data may be provided by a computer and outputted by the computer. Details describing the methods of the computer system are described further in Figure 7 below.

Figure 7 illustrates a block diagram of an example computing system 1500. In some implementations, the computing system 1500 may be utilized in implementing the diagnostic system methods in Figures 3 - 6 below.

In broad overview, the computing system 1510 includes at least one processor 1550 for performing actions in accordance with instructions and one or more memory devices 1570 or 1575 for storing instructions and data. The illustrated example computing system 1510 includes one or more processors 1550 in communication, via a bus 1515, with at least one network interface controller 1520 with network interface ports 1522( a-n) connecting to other computing devices 1524(a-n), memory 1570, and any other devices 1580, e.g., an I/O interface. Generally, a processor 1550 will execute instructions received from memory. The processor 1550 illustrated incorporates, or is directly connected to, cache memory 1575.

In more detail, the processor 1550 may be any logic circuitry that processes instructions, e.g., instructions fetched from the memory 1570 or cache 1575. In many embodiments, the processor 1550 is a microprocessor unit or special purpose processor. The computing device 1500 may be based on any processor, or set of processors, capable of operating as described herein. In some implementations, the processor 1550 can be capable of executing the diagnostic system methods shown in Figures 4-6. The processor 1550 may be a single core or multi-core processor. The processor 1550 may be multiple processors. In some implementations, the processor 1550 can be configured to run multi-threaded operations. In some implementations, the processor 1550 may host one or more virtual machines or containers, along with a hypervisor or container manager for managing the operation of the virtual machines or containers. In such implementations, one or more of the methods 1300 and 1400 shown in Figures 4-6 can be implemented within the virtualized or containerized environments provided on the processor 1550.

The memory 1570 may be any device suitable for storing computer readable data. The memory 1570 may be a device with fixed storage or a device for reading removable storage media. Examples include all forms of non-volatile memory, media and memory devices, semiconductor memory devices (e.g., EPROM, EEPROM, SDRAM, and flash memory devices), magnetic disks, magneto optical disks, and optical discs (e.g., CD ROM, DVD-ROM, and BluRay^{®} discs). A computing system 1500 may have any number of memory devices 1570. In some implementations, the memory 1570 can include instructions corresponding to the Internet notification methods 1300 and 1400 shown in Figures 3 and 4. In some implementations, the memory 1570 supports virtualized or containerized memory accessible by virtual machine or container execution environments provided by the computing system 1510.

The cache memory 1575 is generally a form of computer memory placed in close proximity to the processor 1550 for fast read times. In some implementations, the cache memory 1575 is part of, or on the same chip as, the processor 1550. In some implementations, there are multiple levels of cache 1575, e.g., L2 and L3 cache layers.

The network interface controller 1520 manages data exchanges via the network interfaces 1522(a-n) (also referred to as network interface ports). The network interface controller 1520 handles the physical and data link layers of the OSI model for network communication. In some implementations, some of the network interface controller's tasks are handled by the processor 1550. In some implementations, the network interface controller 1520 is part of the processor 1550. In some implementations, a computing system 1510 has multiple network interface controllers 1520. The network interfaces 1522(a-n) are connection points for physical network links. In some implementations, the network interface controller 1520 supports wireless network connections and an interface port 1522 is a wireless receiver/transmitter. Generally, a computing device 1510 exchanges data with other computing devices 1512(a-n) via physical or wireless links to a network interfaces 1522(a-n). In some implementations, the network interface controller 1520 implements a network protocol such as Ethernet.

The other computing devices 1524(a-n) are connected to the computing device 1510 via a network interface port 1522. The other computing devices 1524(a-n) may be peer computing devices, network devices, or any other computing device with network functionality. For example, a first computing device 1524(a) may be a network device such as a hub, a bridge, a switch, or a router, connecting the computing device 1510 to a data network such as the Internet.

The other devices 1580 may include an I/O interface, external serial device ports, and any additional co-processors. For example, a computing system 1510 may include an interface (e.g., a universal serial bus (USB) interface) for connecting input devices (e.g., a keyboard, microphone, mouse, or other pointing device), output devices (e.g., video display, speaker, or printer), or additional memory devices (e.g., portable flash drive or external media drive). In some implementations, a computing device 1500 includes an additional device 1580 such as a coprocessor, e.g., a math co-processor can assist the processor 1550 with high precision or complex calculations.

Referring back to Figure 1, Figure 3 illustrates a flow chart of an example method for identifying depression biotypes in a subject and predicting a treatment prognosis. The method 1000 includes performing fMRI on a subject (step 1001). The diagnostic system 101 collects the fMRI signal (step 1002). After collecting the fMRI signal, the extraction module 103 within the neuroimaging processor 102 extracts the fMRI BOLD signal 108 (step 1003). The depression biotype classification module 104 receives the extracted fMRI BOLD signal 108 and classifies the subject into an identified depression biotype (step 1004). Additionally, the treatment prognosis classification module 105 receives the identified depression biotype and outputs a treatment prognosis 106 based on the subject's depression biotype 107 (step 1005).

At step 1001, the method 1000 includes performing a resting state fMRI on a subject with an fMRI machine 114. The fMRI machine 114 uses magnetic resonance imaging to map neural networks of a subject's brain and records in vivo a whole brain scan of an individual 115. The resting state fMRI is a method of functional brain imaging that evaluates the connectivity of a subject's brain while the subject is at rest. A resting state subject means that they are awake but not performing any specific task. In some implementations, the subject is laying down with his eyes closed. In some implementations, the subject is laying down with their eyes open. Resting state fMRI is especially useful because it can be easily utilized in diverse patient populations. Resting state fMRI can quantify functional connectivity in resting state brain networks in terms of correlated, spontaneous fluctuations in the MR signal, which are closely related to structural and synaptic measures of connectivity and which are unbiased by the subject performing a specific task.

At step 1002, the neuroimaging processor 102 collects the fMRI signal from the subject. In some implementations, a subject's resting state fMRI includes, but it is not limited to metabolic measures of neural activity mediated by a hemodynamic response. In some implementations, the fMRI signal is preprocessed to control for artifacts caused by the subject's breathing or cardiac pulsations. In some implementations, the fMRI signal is additionally or alternatively preprocessed to control for motion, age, and other scanner related effects in the data.

At step 1003, the neuroimaging processor 102 sends the fMRI signal to the extraction module 103 and the extraction module 103 extracts the fMRI BOLD signal 108 from the fMRI signal. The blood oxygen level dependent signal, or the BOLD signal, measures the increase in blood oxygenation caused by neuronal activity. The fMRI BOLD signal 108 is an indirect measure of neuronal activity. It reflects a function of neural activity, blood flow, and changes in blood volume in the brain. As neurons are stimulated in the brain, oxygenated blood flow increases in the activated region, thereby increasing blood volume. The fMRI BOLD signal 108 measures neural activity through blood oxygen levels.

In some implementation, the extracted fMRI BOLD signal 108 consists of a plurality of connectivity features. The connectivity features represent the large-scale spatial networks of the subject's brain. In some implementations, the extraction module 103 extracts the fMRI BOLD signal 108 after the functional volume of the fMRI data is resampled and co-registered to a common space. In some implementations, a parcellation system is applied to the fMRI data to delineate a plurality of functional networks nodes. In some implementations, the functional network nodes span most cortical, subcortical, and cerebellar areas of the brain. In some implementations, an fMRI BOLD signal residual time series is extracted for each functional node and correlation matrixes are calculated between these time series to obtain an unbiased estimate of the functional connectivity across the whole brain of each subject.

At step 1004, the depression biotype classification module 104 receives the extracted fMRI BOLD signal 108 from step 1003 and classifies the subject into an identified depression biotype 107. As described above, the classification results in an identified depression biotype. Also, as described above, the depression biotype includes one of at least four depression biotypes, Biotype 1, Biotype 2, Biotype 3, and Biotype 4. An identified depression biotype is based on patterns of abnormal connectivity in a subject's resting state brain networks. In some implementations, alterations in fronto-amygdala functional connectivity are most severe in Biotypes 1 and 4. In some implementations, the alterations in thalamic and frontostriatal hyerconnectivity are most severe in Biotypes 3 and 4. In some implementations, alterations in anterior cingulate and orbitofrontal functional connectivity are most severe in Biotypes 1 and 2.

In some implementations, a principal component representation is applied to the functional connectivity features in order to convert the functional connectivity features into linearly uncorrelated variables referred to as principal components. In some implementations, the principal component variables are used as an input for linear support vector machine classifiers to output the likelihood of an identified depression biotype. Details of a method for identifying depression biotypes and details on the alterations in functional connectivity between different brain regions for identifying depression biotypes are described further in relation to Figure 4.

At step 1005, the treatment prognosis classification module 105 uses the depression biotype 107 from step 1004 as an input to classify a subject's likely response to an antidepressant treatment option. The likelihood that a subject will respond to antidepressant treatment is derived from the functional connectivity features extracted from the fMRI signal. In some implementations, a principal component analysis representation is applied to the functional connectivity features. In some implementations, a linear support vector machine classifier calculates a score corresponding to the likelihood of a subject responding to treatment. In some implementations, the antidepressant treatment option is rTMS. A more detailed discussion of methods for classifying a subject's response to a depression treatment option is described in relation to Figure 5.

Referring to Figures 1 and 2, Figure 4 illustrates a flow chart of an example method 2000 for classifying a depression biotype. The method 2000 includes the depression biotype classification module 104 receiving the extracted fMRI BOLD signal 108 from the neuroimaging processor 102 and extraction module 103 (step 2001). The depression biotype classification module measures the functional connectivity using the fMRI BOLD signal 108 from step 2001 (step 2002). Depression biotype classifiers are applied to the pairwise fMRI functional connectivity measures (step 2003). Based on the output of the depression biotype classifiers in step 2003, the subject is identified with a depression biotype (step 2004).

As set forth above, the method 2000 includes receiving the extracted fMRI BOLD signal 108 from the neuroimaging processor 102 and the extraction module 103. The extracted fMRI BOLD signal 108 represents the neural activity in the brain by measuring neural activity through blood oxygen levels.

After the fMRI BOLD signal 108 is extracted from the fMRI signal, the depression biotype classification module 104 measures the subject's functional connectivity (step 2002). In some implementations, the fMRI BOLD signal is extracted after the functional volume of the fMRI data is resampled and co-registered to a common space. In some implementations, a parcellation system may be used to delineate a plurality of functional network nodes spanning most cortical, subcortical, and cerebellar areas of the subject's brain. In some implementations, an fMRI BOLD signal residual time series is extracted from each of the plurality of network nodes and correlation matrixes are performed on these time series to obtain an unbiased estimate of the architecture of functional connectivity across the whole brain in a subject.

A depression biotype classification module 104 collects the subject's depression-related functional connectivity features and classifies the subject into an identified depression biotype (step 2003). As described above, the depression biotype includes one of at least four depression biotypes, Biotype 1, Biotype 2, Biotype 3, and Biotype 4. Each depression biotype relates to distinct patterns of abnormal functional connectivity in the brain. For example, in some implementations, alterations in fronto-amygdala functional connectivity are most severe in Biotypes 1 and 4, which are associated with increased anxiety. In contrast, thalamic and frontostriatal hyperconnectivity are pronounced in Biotype 3 and 4, which are associated with increased anhedonia and psychomotor retardation. Also, anterior cingulate and orbitofrontal connectivity alterations are pronounced in Biotypes 1 and 2, which are associated with increased anergia and fatigue. The depression biotype classifiers may be a linear support vector machine classifiers. The linear support vector machine classifiers are a previously trained machine learning algorithm. In a learning phase, or training phase, the depression biotype classifiers seeks to find a linear optimal hyperplane in order to maximize the separation between multiple classes or multiple biotypes and decipher patterns in the data. In a use phase, the depression biotype classifiers rely on the previously identified patterns to classify the subject into an identified depression biotype 107. In some implementations, the depression biotype classification module 104 may be comprised of four depression biotype classifiers to identify the depression biotype. In some implementations, the depression biotype classification module 104 may be comprised of eight depression biotype classifiers to identify the depression biotype.

The linear support vector machine classifiers identify the subject's depression biotype (step 2004). The linear support vector machine classifiers identify a depression biotype by outputting the likelihood of an identified depression biotype based on the pairwise fMRI functional connectivity measures between neuroanatomical brain regions, described in further detail below. An identified depression biotype is based on patterns of abnormal connectivity in resting state brain networks.

In some implementations, individuals with a known DSM (Diagnostic and Statistical Manual of Mental Disorder) diagnosis may be classified into one of the four depression biotypes Biotype 1, Biotype 2, Biotype 3, and Biotype 4. For example, when an individual has a known DSM diagnosis, the depression biotype classification module may be comprised of four depression biotype classifiers, one for each of the four depression biotypes. Each depression biotype classifier is a linear support vector machine classifier. Each of the four depression biotype classifiers calculates a score corresponding to the likelihood of a given individual fitting within its respective biotype. The depression biotype classification module 104 then assigns the individual to an identified depression biotype with the highest classifier score. The classifier scores derive from the pairwise fMRI functional connectivity measures between the following neuroanatomical brain regions including, dorsomedial prefrontal cortex, middle temporal gyrus, parahippocampal cortex, dorsolateral prefrontal cortex, anterior prefrontal cortex, posterior parietal cortex, anterior cingulate cortex, insular cortex, ventrolateral prefrontal cortex, precuneus, orbitofrontal cortex, ventral striatum thalamus, raphe nucleus (midbrain), primary cortex, somatosensory cortex, and visual cortex.

In some implementations, individuals with no known DSM diagnosis may be classified into one of the four depression biotypes. For example, when an individual has no known DSM diagnosis, the depression biotype classification module may be comprised of eight linear support vector machine classifiers. Four of the linear support vector machine classifiers correspond to each of the four depression biotypes and the remaining four linear support vector machine classifiers correspond to whether the individual is depressed or healthy. First, the depression biotype classification module 104 assigns the individual to an identified depression biotype based on their highest score from the four depression biotype classifiers. As mentioned above, the score for each of the biotype classifiers may be derived from pairwise fMRI functional connectivity measures between the following neuroanatomical brain regions including, dorsomedial prefrontal cortex, middle temporal gyrus, parahippocampal cortex, dorsolateral prefrontal cortex, anterior prefrontal cortex, posterior parietal cortex, anterior cingulate cortex, insular cortex, ventrolateral prefrontal cortex, precuneus, orbitofrontal cortex, ventral striatum thalamus, raphe nucleus (midbrain), primary cortex, somatosensory cortex, and visual cortex. Second, the depression biotype classification module 104 determines whether the individual belongs in that identified depression biotype or is not actively depressed, as described below.

In some implementations, the classifier determining whether the individual belongs in Biotype 1 or is not actively depressed may be derived from pairwise fMRI functional connectivity measures between the following neuroanatomical brain regions including, posterior parietal cortex, precuneus, middle temporal gyrus, parahippocampal cortex, dorsolateral prefrontal cortex, ventrolateral prefrontal cortex, insular cortex, temporal pol, superior temporal gyrus, ventromedial prefrontal cortex, ventral hippocampus, amygdala, ventral caudate nucleus, ventral striatum, thalamus, primary somatosensory cortex, primary motor cortex, visual cortex.

In some implementations, the classifier determining whether the individual belongs in Biotype 2 or is not actively depressed may be derived from pairwise fMRI functional connectivity measures between the following neuroanatomical brain regions including, dorsomedial prefrontal cortex, ventromedial prefrontal cortex, orbitofrontal cortex, posterior cingulate cortex, fusiform gyrus, middle temporal gyrus, parahippocampal cortex, anterior cingulate cortex, ventrolateral prefrontal cortex, dorsolateral prefrontal cortex, insular cortex, posterior parietal cortex, thalamus, primary somatosensory cortex, primary motor cortex, and visual cortex.

In some implementations, the classifier determining whether the individual belongs in Biotype 3 or is not actively depressed may be derived from pairwise fMRI functional connectivity measures between the following neuroanatomical brain regions including, ventromedial prefrontal cortex, posterior parietal cortex, middle temporal gyrus, ventrolateral prefrontal cortex, anterior prefrontal cortex, dorsolateral prefrontal cortex, posterior parietal cortex, temporal pole, superior temporal gyrus, orbitofrontal cortex, parahippocampal cortex, ventral hippocampus, amygdala, subgenual anterior cingulate cortex, ventral caudate nucleus, ventral striatum, thalamus, premotor cortex, supplementary motor area, insular cortex, anterior cingulate cortex, fusiform gyrus.

In some implementations, the classifier determining whether the individual belongs in Biotype 4 or is not actively depressed may be derived from pairwise fMRI functional connectivity measures between the following neuroanatomical brain regions including, fusiform gyrus, parahippocampal cortex, ventrolateral prefrontal cortex, posterior parietal cortex, anterior cingulate cortex, middle cingulate cortex, insular cortex, temporal pole, superior temporal gyrus, middle temporal gyrus, orbitofrontal cortex, ventral hippocampus, subgenual anterior cingulate cortex, ventral striatum, thalamus, primary somatosensory cortex, supplementary motor area, insular cortex, postcentral gyrus, and lingual gyrus.

In some implementations, the classifier score may be derived based on the pairwise fMRI functional connectivity measures of all of the above brain regions. In some implementations, the classifier score may be derived based on a subset of the pairwise fMRI functional connectivity measures of the above brain regions. In some implementations, the classifier may be based on pairwise fMRI functional connectivity measures between the above mentioned brain regions with additional brain regions or between pairs of other brain regions neither of which are mentioned above. In some implementations, the fMRI measures of functional connectivity are taken between the centroids of a pair of neuroanatomical brain regions. In some implementations, the fMRI measures of functional connectivity are taken between corresponding points within the vicinity of the respective neuroanatomical brain regions of a given pair of regions.

In some implementations, the score for each classifier may be derived from a low-dimensional principal component representation of the functional connectivity between the neuroanatomical brain regions listed above, plus a patient's clinical symptoms. The principal component analysis representation is a decomposition method used to reduce the dimensionality of data. The principal component analysis representation of the subject's functional connectivity features reduces the number of features used to represent the data by applying an orthogonal transformation to convert the functional connectivity features into a set of linearly correlated variables called principal components. The number of principal components is less than the number of functional connectivity features. As would be known to a person having ordinary skill in the art, principal component analysis representation is frequently used for data compression and feature selection. The patient's clinical symptoms may be represented by either a self-reported clinical symptom rating such as the patient's item-level responses to the 21-item Beck Depression Inventory, or clinician rating of clinical symptom severity such as the patient's item-level responses to the 17-item Hamilton Depression Rating Scale ("Ham-D").

Referring to Figure 2, Figure 5 illustrates a flow chart of an example method 3000 for classifying a subject's likely treatment response based on their identified depression biotype. The treatment prognosis classification module 109 identifies the treatment prognosis 106 based on the connectivity features and the clinical symptom profile to differentiate treatment responders from non-responders. It outputs the likelihood that the subject will respond to an antidepressant treatment. The method 3000 includes performing fMRI imaging on a subject by using an fMRI machine 114 (step 3001). The fMRI BOLD signal 108 is extracted from the fMRI data (step 3002) and the depression biotype is identified (step 3003). The treatment prognosis classification module 109 predicts the subject's antidepressant response to treatment based on the subject's pairwise fMRI functional connectivity measures (step 3004).

As set forth above, the method 3000 includes performing a resting state fMRI on a subject through an fMRI machine 114 (step 3001). The step 3001 can be identical to step 1001 shown before.

A neuroimaging processor 102 collects the fMRI data from the subject and the extraction module 103 extracts the fMRI BOLD signal 108 (step 3002). As mentioned above, the fMRI BOLD signal is an indirect measurement of neural activity in the brain of the subject. The step 3002 can be identical to step 1003 shown before.

Next, the method 3000 includes identifying the individual's depression biotype (step 3003). As described above, the depression biotype includes one of at least four depression biotypes, Biotype 1, Biotype 2, Biotype 3, and Biotype 4. Each depression biotype relates to distinct patterns of abnormal functional connectivity in the brain. In step 3003, the treatment prognosis classification module 109 receives the fMRI BOLD signal 108 and identifies the individual's depression biotype. Similar to above, in some implementations, individuals with a known DSM (Diagnostic and Statistical Manual of Mental Disorder) diagnosis may be classified into one of the four depression biotypes. For example, when an individual has a known DSM diagnosis, the treatment prognosis classification module 109 may be comprised of four linear support vector machine classifiers, one for each of the four depression biotypes. Each of the four depression biotype classifiers calculates a score corresponding to the likelihood of a given individual fitting within each of the four biotypes. The treatment prognosis classification module 109 then assigns the individual to an identified depression biotype with the highest classifier score. In some implementations, each classifier score may be derived from pairwise fMRI functional connectivity measures between the following neuroanatomical brain regions including, dorsomedial prefrontal cortex, middle temporal gyrus, parahippocampal cortex, dorsolateral prefrontal cortex, anterior prefrontal cortex, posterior parietal cortex, anterior cingulate cortex, insular cortex, ventrolateral prefrontal cortex, precuneus, orbitofrontal cortex, ventral striatum thalamus, raphe nucleus (midbrain), primary cortex, somatosensory cortex, and visual cortex.

In some implementations, the treatment prognosis classification module 109 may classify individuals with no known DSM diagnosis into one of the four depression biotypes. For example, when an individual has no known DSM diagnosis, the treatment prognosis classification module 109 may be comprised of eight linear support vector machine classifiers. Four of the linear support vector machine classifiers correspond to each of the four depression biotypes and the remaining four linear support vector machine classifiers correspond to each of the four depression biotypes and whether the individual is actively depressed. First, the treatment prognosis classification module 109 assigns the individual to an identified depression biotype based on the highest score. As mentioned above, the score for each of the biotype classifiers may be derived from pairwise fMRI functional connectivity measures between the following neuroanatomical brain regions including, dorsomedial prefrontal cortex, middle temporal gyrus, parahippocampal cortex, dorsolateral prefrontal cortex, anterior prefrontal cortex, posterior parietal cortex, anterior cingulate cortex, insular cortex, ventrolateral prefrontal cortex, precuneus, orbitofrontal cortex, ventral striatum thalamus, raphe nucleus (midbrain), primary cortex, somatosensory cortex, and visual cortex. Second, the prognosis classification module 109 determines whether the individual belongs in that identified depression biotype or is not actively depressed, as described in the following four sections.

In some implementations, the classifier determining whether the individual belongs in Biotype 1 or is not actively depressed may be derived from pairwise fMRI functional connectivity measures between the following neuroanatomical brain regions including, posterior parietal cortex, precuneus, middle temporal gyrus, parahippocampal cortex, dorsolateral prefrontal cortex, ventrolateral prefrontal cortex, insular cortex, temporal pol, superior temporal gyrus, ventromedial prefrontal cortex, ventral hippocampus, amygdala, ventral caudate nucleus, ventral striatum, thalamus, primary somatosensory cortex, primary motor cortex, visual cortex.

In some implementations, the classifier determining whether the individual belongs in Biotype 2 or is not actively depressed may be derived from pairwise fMRI functional connectivity measures between the following neuroanatomical brain regions including, dorsomedial prefrontal cortex, ventromedial prefrontal cortex, orbitofrontal cortex, posterior cingulate cortex, fusiform gyrus, middle temporal gyrus, parahippocampal cortex, anterior cingulate cortex, ventrolateral prefrontal cortex, dorsolateral prefrontal cortex, insular cortex, posterior parietal cortex, thalamus, primary somatosensory cortex, primary motor cortex, and visual cortex.

In some implementations, the classifier determining whether the individual belongs in Biotype 3 or is not actively depressed may be derived from pairwise fMRI functional connectivity measures between the following neuroanatomical brain regions including, ventromedial prefrontal cortex, posterior parietal cortex, middle temporal gyrus, parahippocampal cortex, ventrolateral prefrontal cortex, anterior prefrontal cortex, dorsolateral prefrontal cortex, posterior parietal cortex, temporal pole, superior temporal gyrus, orbitofrontal cortex, ventral hippocampus, amygdala, subgenual anterior cingulate cortex, ventral caudate nucleus, ventral striatum, thalamus, premotor cortex, supplementary motor area, insular cortex, anterior cingulate cortex, fusiform gyrus.

In some implementations, the classifier determining whether the individual belongs in Biotype 4 or is not depressed may be derived from pairwise fMRI functional connectivity measures between the following neuroanatomical brain regions including, fusiform gyrus, parahippocampal cortex, ventrolateral prefrontal cortex, posterior parietal cortex, anterior cingulate cortex, middle cingulate cortex, insular cortex, temporal pole, superior temporal gyrus, middle temporal gyrus, orbitofrontal cortex, ventral hippocampus, subgenual anterior cingulate cortex, ventral striatum, thalamus, primary somatosensory cortex, supplementary motor area, insular cortex, postcentral gyrus, and lingual gyrus.

Next, the treatment prognosis classifier outputs the likelihood that the subject will respond to an antidepressant treatment (step 3004). Similar to the depression biotype classifiers, the treatment prognosis classifier may be a linear support vector machine classifier. The linear support vector machine classifier is a previously trained machine learning algorithm. In the learning phase, or training phase, the treatment prognosis classifier seeks to find an optimal hyperplane in order to maximize the separation between multiple classes or treatment outcomes and decipher patterns in the data. In the use phase, the treatment prognosis classifier relies on the previously identified patterns to classify the subject into an identified treatment prognosis. The treatment prognosis classifier calculates a score corresponding to the likelihood that the individual will respond to antidepressant treatment. The score for the treatment prognosis classifier may be derived from the identified depression biotype of step 3003 and the pairwise fMRI functional connectivity measures. In some implementations, pairwise fMRI functional connectivity measures may be derived from the following neuroanatomical brain regions including, dorsomedial prefrontal cortex, anterior cingulate cortex, posterior cingulate cortex, ventromedial prefrontal cortex, ventrolateral prefrontal cortex, dorsolateral prefrontal cortex, posterior parietal cortex, orbitofrontal cortex, amygdala, ventral striatum, nucleus accumbens, globus pallidus, thalamus, primary motor cortex, primary somatosensory cortex, and visual cortex.

In some implementations, the classifier score may be derived based on the pairwise fMRI functional connectivity measures of all of the above brain regions. In some implementations, the classifier score may be derived based on a subset of the pairwise fMRI functional connectivity measures of the above brain regions. In some implementations, the classifier may be based on pairwise fMRI functional connectivity measures between the above mentioned brain regions with additional brain regions or between pairs of other brain regions neither of which are mentioned above. In some implementations, the fMRI measures of functional connectivity are taken between the centroids of a pair of neuroanatomical brain regions. In some implementations, the fMRI measures of functional connectivity are taken between corresponding points within the vicinity of the respective neuroanatomical brain regions of a given pair of regions.

In some implementations, the score from treatment prognosis classifier may be derived from a principal component representation. The principal component analysis representation is a decomposition method used to reduce the dimensionality of data. The principal component analysis representation of the functional connectivity features reduces the number of features used to represent the data. The score for the treatment prognosis classifier may be derived from the identified depression biotype from step 3003 and the principal component representation of the same fMRI measure of functional connectivity derived from the neuroanatomical brain regions listed above. In some implementations, the principal component representation of the subject's functional connectivity features in the treatment prognosis module also applies the subject's clinical symptom profile of depression. In some implementations, the clinical symptom profiles include a 17 item Ham-D clinician-administered depression assessment scale. In some implementations, the clinical symptom profile includes a 21 item Beck Depression Inventory self-reported depression assessment scale.

Testing for treatment response prediction is an important element of validating novel biomarkers and establishing their utility and potential for clinical actionability. In some implementations, the antidepressant treatment is a non-invasive neurostimulation treatment of rTMS targeting the dorsomedial prefrontal cortex for depression. rTMS is a non-invasive neurostimulation treatment for medication-resistant depression that acts by enhancing synaptic plasticity at the prefrontal stimulation target and modulating functional connectivity in cortical networks. In some implementations, efficacy of rTMS varies with functional connectivity measures. For example, the left dorsolateral prefrontal cortex is the most common target for stimulation, but efficacy also exists for a dorsomedial prefrontal cortical target. In some implementations, the dorsomedial prefrontal cortical area may be among some of the important neuroanatomical areas differentiating the four biotypes. Individual differences in the antidepressant response to rTMS may be predicted on the basis of the differences in functional connectivity and symptom profiles. In some implementations, patients in Biotype 1 were more than three times more likely to benefit from transcranial magnetic stimulation of the dorsomedial prefrontal cortex than those in Biotypes 2 or 4.

Referring to Figures 1 and 2, Figure 6 illustrates a flow chart of an example method for predicting a subject's depression severity and tracking it over time. The severity module 120 quantifies the severity of a patient's depression based on the fMRI data instead of patient self-reports. The severity module 120 outputs the patient's depression severity to the severity tracker 121, which tracks the patient's change in depression severity over time. The method 4000 includes performing resting state fMRI imaging on a subject using an fMRI machine 114 (step 4001). The fMRI BOLD signal is extracted from the fMRI data (step 4002). The severity module 120 receives the fMRI BOLD signal from the neuroimaging processor 102 and predicts the subject's depression severity (step 4003). The severity module 120 outputs the subject's depression severity to the severity tracker and the severity tracker follows the subject's severity over time through multiple fMRI scans (step 4004).

As set forth above, the method 4000 includes performing a resting state fMRI on a subject through an fMRI machine 114 (step 4001). The fMRI machine uses magnetic resonance imaging to map neural networks of a subject's brain and records in vivo a whole brain scan of an individual. The resting state fMRI is a method of functional brain imaging that evaluates the connectivity of a subject's brain while the subject is at rest. The step 4001 can be identical to step 1001 shown above in Figure 3.

A neuroimaging processor 102 collects the fMRI data from the subject and the extraction module 103 extracts the fMRI BOLD signal 108 (step 4002). As mentioned above, the fMRI BOLD signal is an indirect measurement of neural activity. The step 4002 can be identical to step 1003 shown above in Figure 3.

The severity module 120 utilizes a multiple linear regression model to predict a subject's depression severity (step 4003). Multiple linear regression is a statistical method that studies the relationship between a response variable and multiple continuous, quantitative predictor variables. The response variable may be depression severity and the multiple continuous, quantitative predictor variables may be pairwise fMRI functional connectivity measures. In some implementations, the response variable may represent the actual depression severity of the subject, and the predictor variables may represent the pairwise fMRI functional connectivity measures. The multiple linear regression model models the relationship between multiple predictor variables and a response variable by fitting a linear equation to the observed data. The multiple linear regression model predicts depression severity by assigning weights to pairwise fMRI functional connectivity measures and summing across these weighted measures to yield a predicted depression severity score. The severity score positively correlates with a patient's depression severity. In some implementations, the multiple linear regression model utilizes pairwise fMRI functional connectivity measures between the following neuroanatomical brain regions including, dorsomedial prefrontal cortex, posterior cingulate cortex, precuneus, posterior parietal cortex, middle temporal gyrus, parahippocampal cortex, anterior prefrontal cortex, anterior cingulate cortex, ventrolateral prefrontal cortex, insular cortex, orbitofrontal cortex, ventral hippocampus, ventral striatum, thalamus, primary motor cortex, premotor cortex, and visual cortex. In some implementations, the severity module may include a low dimensional principal component representation of the pairwise fMRI functional connectivity measures.

In some implementations, the classifier score may be derived based on the pairwise fMRI functional connectivity measures of all of the above brain regions. In some implementations, the classifier score may be derived based on a subset of the pairwise fMRI functional connectivity measures of the above brain regions. In some implementations, the classifier may be based on pairwise fMRI functional connectivity measures between the above mentioned brain regions with additional brain regions or between pairs of other brain regions neither of which are mentioned above. In some implementations, the fMRI measures of functional connectivity are taken between the centroids of a pair of neuroanatomical brain regions. In some implementations, the fMRI measures of functional connectivity are taken between corresponding points within the vicinity of the respective neuroanatomical brain regions of a given pair of regions.

Next, the method 4000 includes outputting the subject's predicted depression severity score to the severity tracker 121. The severity tracker receives the subject's predicted depression severity score and stores the information. Over time and based on multiple resting state fMRI scans, the severity tracker compiles the subject's predicted severity depression scores. In comparison to evaluating depression severity based on answers to questionnaires, as is the typical practice currently, the fMRI-based severity depression scores are a more objective means to evaluate a subject's depression severity. For example, such scores are not susceptible to errors due to inconsistent interpretations of questionnaire questions or potential inaccurate or otherwise non-representative responses from subjects answering the questionnaires. In some implementations, the depression severity scores may be used for language or otherwise developmentally impaired or disabled subjects who may not be competent or capable of effectively responding to the questionnaires. In some implementations, the severity tracker 121 can determine if the subject's compilation of predicted severity scores increased or decreased, which determines whether the subject's depression severity has increased or decreased over time. In some implementation, the severity tracker 121 can determine the antidepressant treatment effectiveness based on changes over time in the subject's compilation of predicted severity scores. In some implementations, the severity tracker 121 can track the depression severity scores across multiple subjects having been identified as having the same depression biotype and receiving the same treatment, for example in the context of a clinical trial, to assess the effectiveness of the clinical trial.

### Experimental Results

To support the techniques described above, experiments were conducted to identify parameters that are particularly effective to determine depression biotypes and facilitate biotype classification. The biotype cluster discovery data was collected from 220 patients. To ensure that the cluster discovery was not confounded by site-related differences in subject recruitment criteria or other undefined variables, the cluster discovery analysis was conducted on these 220 patients who were recruited from two sites, with identical inclusion and exclusion criteria and statistically equivalent depression symptom scores.

The subjects underwent an fMRI scan of their brain. The data was processed to control for motion, age, and other scanner related effects. After resampling and co-registering the functional volumes to common space, a parcellation system was applied to the fMRI data to delineate 258 functional network nodes. Next, the fMRI BOLD signal residual time series was extracted for each functional node and correlation matrixes were calculated between these time series to obtain an unbiased estimate of the architecture of functional connectivity across the whole brain.

Each correlation matrix comprised about 33,000 unique connectivity features. To select a subset of connectivity features, the processor applied a canonical correlation analysis associated with weighted combinations of clinical symptoms, as quantified by the 17 item Ham-D scale, to the extracted fMRI BOLD signal data. The analysis revealed linear combinations of connectivity features (analogous to principal components) that predicted two distinct sets of depressive symptoms.

The first connectivity feature defined a combination of predominantly frontostriatal and orbitofrontal connectivity features that were correlated with anhedonia and psychomotor retardation. The second connectivity feature defined a distinct set of predominantly limbic connectivity features involving the amygdala, ventral hippocampus, ventral striatum, subgenual cingulate, and later prefrontal control areas and correlated with anxiety and insomnia. Hierarchical clustering revealed four depression biotypes defined by distinct and relatively homogenous patterns of connectivity along these two connectivity components. Additional potential clustering solutions were also evident within these four groups. In some implementations, the hierarchical cluster analysis uses Ward's minimum variance method.

After applying Wilcoxon rank sum tests and Kruskal Wallis ANOVA to the connectivity features of each biotype, two themes were revealed. First, a common neuroanatomical core of pathology underlying all four biotypes existed, which spanned the insula, orbitofrontal cortex, ventromedial prefrontal cortex, and multiple subcortical areas. The common neuroanatomical core predicted the severity of three "core" symptoms (depressed mood, anhedonia, fatigue) that were present in almost all patients, greater than 93.9%. Across all subjects, regardless of biotype, abnormal connectivity in the shared neuroanatomical core was significantly correlated with the three "core" depression symptoms between r = 0.72 and r = 0.82.

Also, distinct patterns of abnormal functional connectivity differentiated the four biotypes and were associated with specific clinical symptom profiles. For example, alterations in fronto-amygdala functional connectivity were most severe in Biotypes 1 and 4, which were associated with increased anxiety, a finding consistent with known roles for these projections in regulating fear-related behavior and reappraising the affective salience of negative emotional stimuli. In contrast, thalamic and frontostriatal hyperconnectivity were especially pronounced in Biotypes 3 and 4, and were associated with increased anhedonia and psychomotor retardation, in accord with posited roles for these projections in reward processing, adaptive motor control, and initiating movements. Anterior cingulate and orbitofrontal connectivity alterations were most severe in Biotypes 1 and 2, and were associated with increased anergia and fatigue, consistent with the role of these circuits in motivation and incentive salience assessments, respectively. These findings delineate clinically meaningful, homogeneous biotypes of depression with robust neurobiological correlates defined by distinct patterns of abnormal functional connectivity in frontostriatal and limbic networks, superimposed on one shared core pathology.

For developing classifiers for diagnosing depression biotypes based on pairwise fMRI functional connectivity measures, the classification process was optimized in a training dataset comprising 333 patients and 378 healthy controls matched for age and sex. The classifiers were trained iteratively on a randomly selected subset of these subjects, and then tested on an independent subset left out of all aspects of the training process. Support vector machine classifiers yielded overall accuracy rates of up to 89.2% based on connectivity features with the neuroanatomical areas, listed above in Figure 4. In some implementations, the pairwise fMRI functional connectivity measures may include dorsomedial prefrontal cortex, middle temporal gyrus, parahippocampal cortex, dorsolateral prefrontal cortex, anterior prefrontal cortex, posterior parietal cortex, anterior cingulate cortex, insular cortex, ventrolateral prefrontal cortex, precuneus, orbitofrontal cortex, ventral striatum thalamus, raphe nucleus (midbrain), primary cortex, somatosensory cortex, and visual cortex. In some implementations, the pairwise fMRI functional connectivity measures may include posterior parietal cortex, precuneus, middle temporal gyrus, parahippocampal cortex, dorsolateral prefrontal cortex, ventrolateral prefrontal cortex, insular cortex, temporal pol, superior temporal gyrus, ventromedial prefrontal cortex, ventral hippocampus, amygdala, ventral caudate nucleus, ventral striatum, thalamus, primary somatosensory cortex, primary motor cortex, visual cortex. In some implementations, the pairwise fMRI functional connectivity features may include dorsomedial prefrontal cortex, ventromedial prefrontal cortex, orbitofrontal cortex, posterior cingulate cortex, fusiform gyrus, middle temporal gyrus, parahippocampal cortex, anterior cingulate cortex, ventrolateral prefrontal cortex, dorsolateral prefrontal cortex, insular cortex, posterior parietal cortex, thalamus, primary somatosensory cortex, primary motor cortex, and visual cortex. In some implementations, the pairwise fMRI functional connectivity measures may include ventromedial prefrontal cortex, posterior parietal cortex, middle temporal gyrus, ventrolateral prefrontal cortex, anterior prefrontal cortex, dorsolateral prefrontal cortex, posterior parietal cortex, temporal pole, superior temporal gyrus, orbitofrontal cortex, parahippocampal cortex, ventral hippocampus, amygdala, subgenual anterior cingulate cortex, ventral caudate nucleus, ventral striatum, thalamus, premotor cortex, supplementary motor area, insular cortex, anterior cingulate cortex, fusiform gyrus. In some implementations, the pairwise fMRI functional connectivity measures may include fusiform gyrus, parahippocampal cortex, ventrolateral prefrontal cortex, posterior parietal cortex, anterior cingulate cortex, middle cingulate cortex, insular cortex, temporal pole, superior temporal gyrus, middle temporal gyrus, orbitofrontal cortex, ventral hippocampus, subgenual anterior cingulate cortex, ventral striatum, thalamus, primary somatosensory cortex, supplementary motor area, insular cortex, postcentral gyrus, and lingual gyrus.

For further biotype validation, the classifiers were tested on a subset of patients, N=50, who received a second fMRI scan wile actively depressed, 4 - 6 weeks after the first scanning session. The results showed that 90% of subjects were assigned to the same biotype in both scans, χ2=84.6, p < 0.0001, including 92.3% and 93.3% of subjects in Biotypes 2 and 3. Tests were also conducted to determine whether the clustering results were influenced by differences in other variables that have known or suspected effects on resting state fMRI measures of functional connectivity, including age, medication usage, and head motion during scanning. Results showed no significant between group differences on any of these measures.

To ensure that the classifier performance was not an overestimation based on iterative training and cross-validation on the same data, further tests were conducted. The most successful classifier for each biotype was tested on an independent replication dataset, comprising 125 patients and 352 healthy controls acquired from 13 sites, including 5 sites that were not included in the original training dataset. To avoid overestimating diagnostic sensitivity, only one classifier, the classifier for the best fitting biotype, was tested on each subject, using the same two-step process outlined in Paragraphs 45-49 above. Overall, 86.2% of subjects in this independent, out-of-sample replication dataset were correctly diagnosed, including more than 90% of patients in Biotypes 3 and 4. By implementing stricter data quality controls and by treating subjects with ambiguous classification outcomes as equivocal test results (i.e. the lowest absolute SVM classification scores), these accuracy rates increased to more than 95%. This finding suggests room for improved diagnostic performance for neuroimaging biomarkers in prospective datasets acquired using state-of-the-art technology, which has proven capable of delivering significant improvements in signal quality and stability.

To test whether biotype differences in dysfunctional connectivity at the dorsomedial prefrontal cortical target site may give rise to differing treatment outcomes, a total of 124 subjects received repetitive high-frequency stimulation of the dorsomedial prefrontal cortex for five weeks beginning shortly after their fMRI scan. Treatment response varied significantly with cluster membership (χ2=25.7, p=1.1x10-5). rTMS was most effective for patients in Biotype 1, 82.5% of whom (33 of 40 subjects) improved significantly after treatment (partial response=25+% decrease in severity by Ham-D), compared to 61.0% for Biotype 3 (25 of 41 subjects) and only 25.0% and 29.6% for Biotypes 2 (4 of 16 subjects) and 4 (8 of 27 subjects), respectively.

The classifiers were trained to differentiate responders and non-responders using the same approach to feature selection, training, and leave-one-out cross-validation as described above. This tested whether connectivity based biotypes could be used to predict treatment response more effectively than clinical symptoms alone. The most discriminating functional connectivity features involved the dorsomedial prefrontal stimulation target and the left amygdala, left dorsolateral prefrontal cortex, bilateral orbitofrontal cortex, and posterior cingulate cortex. Connectivity between other neuroanatomical areas that were not directly stimulated by the rTMS protocol-including the ventromedial prefrontal cortex, thalamus, nucleus accumbens, and globus pallidus-was also highly associated with subsequent treatment response. Individual differences in the antidepressant response to rTMS could be predicted on the basis of these differences in functional connectivity with 78.3% accuracy in leave-one-out cross-validation analysis. Biotype diagnosis significantly improved classifier performance. Classification based on the same discriminating connectivity features plus biotype diagnosis increased overall accuracy rates to 89.6%, with a 93.6% correct prediction rate for non-responders, p < 0.001. Accuracy rates could be further improved from 89.6% to >94% by implementing stricter data quality controls and treating subjects with ambiguous classification outcomes as equivocal test results.

In contrast, clinical symptoms alone were not strong predictors of rTMS treatment responsiveness at an individual level. To test this, classifiers were trained to differentiate responders and non-responders based solely on clinical data. The clinical features alone using Ham-D item-level responses were only modestly predictive of treatment responsiveness with 62.6% overall accuracy.

To further evaluate predictive validity, the best performing classifier, which utilized a combination of connectivity features and biotype diagnosis, was tested in an independent replication set, N=30 subjects, and obtained comparable accuracy rates: 87.5% for all subjects and 92.6% for subjects with high data quality and unequivocal predicted outcomes Supplementary analyses provide additional evidence that clinical symptom measures alone cannot substitute for resting state fMRI measures: subtyping subjects based on clinical symptoms yielded highly variable, longitudinally unstable clustering outcomes that also failed to predict treatment response. These results provide strong convergent evidence that a data-driven approach to clustering patients based on functional connectivity measures revealed biologically meaningful biotypes of depression with robust neurobiological correlates and strong predictive validity for a neurostimulatory antidepressant targeting dysfunction in the dorsomedial prefrontal cortical area.

To test whether a subject's depression severity can be determined based on brain region functional connectivity measures, a multiple linear regression model was fit to a sample of N=497 patients with major depressive disorder. A clinician-rated clinical symptom severity using the 17-item Ham-D depression scale scored patient severity. The score in this sample (N=497) ranged from 4, i.e., patients in full remission, to 38, i.e., patients with severe depression. The mean Ham-D score was 22.5 and the standard deviation was 5.2. A multiple linear regression model predicted a subject's depression severity by assigning weights to pairwise fMRI functional connectivity measures and summing across these weighted measures to yield a predicted depression severity. The multiple linear regression model utilized pairwise fMRI functional connectivity measures between the following neuroanatomical brain regions including, dorsomedial prefrontal cortex, posterior cingulate cortex, precuneus, posterior parietal cortex, middle temporal gyrus, parahippocampal cortex, anterior prefrontal cortex, anterior cingulate cortex, ventrolateral prefrontal cortex, insular cortex, orbitofrontal cortex, ventral hippocampus, ventral striatum, thalamus, primary motor cortex, premotor cortex, and visual cortex. The method enabled accurate prediction of depression severity (root mean squared error = 4.2 Ham-D points) based on brain region functional connectivity features, accounting for 48.5% of the variance in depression severity in the sample of N=497 depressed patients.

Implementations of the subject matter and the operations described in this specification can be implemented in digital electronic circuitry, or in computer software embodied on a tangible medium, firmware, or hardware, including the structures disclosed in this specification and their structural equivalents, or in combinations of one or more of them. Implementations of the subject matter described in this specification can be implemented as one or more computer programs embodied on a tangible medium, i.e., one or more modules of computer program instructions, encoded on one or more computer storage media for execution by, or to control the operation of, a data processing apparatus. A computer storage medium can be, or be included in, a computer-readable storage device, a computer-readable storage substrate, a random or serial access memory array or device, or a combination of one or more of them. The computer storage medium can also be, or be included in, one or more separate components or media (e.g., multiple CDs, disks, or other storage devices). The computer storage medium may be tangible and non-transitory.

The operations described in this specification can be implemented as operations performed by a data processing apparatus on data stored on one or more computer-readable storage devices or received from other sources. The operations may be executed within the native environment of the data processing apparatus or within one or more virtual machines or containers hosted by the data processing apparatus.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers or one or more virtual machines or containers that are located at one site or distributed across multiple sites and interconnected by a communication network. Examples of communication networks include a local area network ("LAN") and a wide area network ("WAN"), an inter-network (e.g., the Internet), and peer-to-peer networks (e.g., ad hoc peer-to-peer networks).

The processes and logic flows described in this specification can be performed by one or more programmable processors executing one or more computer programs to perform actions by operating on input data and generating output. The processes and logic flows can also be performed by, and apparatus can also be implemented as, special purpose logic circuitry, e.g., an FPGA (field programmable gate array) or an ASIC (application specific integrated circuit).

While this specification contains many specific implementation details, these should not be construed as limitations on the scope of any inventions or of what may be claimed, but rather as descriptions of features specific to particular implementations of particular inventions. Certain features that are described in this specification in the context of separate implementations can also be implemented in combination in a single implementation. Conversely, various features that are described in the context of a single implementation can also be implemented in multiple implementations separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the implementations described above should not be understood as requiring such separation in all implementations, and it should be understood that the described program components and systems can generally be integrated together in a single software product or packaged into multiple software products.

References to "or" may be construed as inclusive so that any terms described using "or" may indicate any of a single, more than one, and all of the described terms. The labels "first," "second," "third," and so forth are not necessarily meant to indicate an ordering and are generally used merely to distinguish between like or similar items or elements.

Various modifications to the implementations described in this disclosure may be readily apparent to those skilled in the art, and the generic principles defined herein may be applied to other implementations without departing from the scope of this disclosure. Thus, the claims are not intended to be limited to the implementations shown herein, but are to be accorded the widest scope consistent with this disclosure, the principles and the novel features disclosed herein.

## Claims

1. A method for identifying a neurophysiological biotype of depression in the brain of a patient, comprising:
receiving, by a processor, recorded fMRI data indicative of brain activity of the patient;
extracting, by the processor, brain region functional connectivity information from the fMRI signal;
identifying a depression biotype for the patient by applying a biotype classifier executing on the processor to the brain region functional connectivity information extracted from the fMRI signal;
outputting, by the processor, the identified depression biotype,
wherein determining if the patient suffers from one of a Biotype 1, a Biotype 2, a Biotype 3 or a Biotype 4 depression is derived from pairwise fMRI functional connectivity measures between different neuroanatomical brain regions,
for a Biotype 1 depression from pairwise fMRI functional connectivity measures between neuroanatomical brain regions including the posterior parietal cortex, precuneus, middle temporal gyros, parahippocampal cortex, dorsolateral prefrontal cortex, ventrolateral prefrontal cortex, insular cortex, temporal pol, superior temporal gyros, ventromedial prefrontal cortex, ventral hippocampus, amygdala, ventral caudate nucleus, ventral striatum, thalamus, primary somatosensory cortex, primary motor cortex, visual cortex,
for a Biotype 2 depression from pairwise fMRI functional connectivity measures between neuroanatomical brain regions including the dorsomedial prefrontal cortex, ventromedial prefrontal cortex, orbitofrontal cortex, posterior cingulate cortex, fusiform gyros, middle temporal gyros, parahippocampal cortex, anterior cingulate cortex, ventrolateral prefrontal cortex, dorsolateral prefrontal cortex, insular cortex, posterior parietal cortex, thalamus, primary somatosensory cortex, primary motor cortex, and visual cortex,
for a Biotype 3 depression from pairwise fMRI functional connectivity measures between neuroanatomical brain regions including the ventromedial prefrontal cortex, posterior parietal cortex, middle temporal gyros, ventrolateral prefrontal cortex, anterior prefrontal cortex, dorsolateral prefrontal cortex, posterior parietal cortex, temporal pole, superior temporal gyros, orbitofrontal cortex, parahippocampal cortex, ventral hippocampus, amygdala, subgenual anterior cingulate cortex, ventral caudate nucleus, ventral striatum, thalamus, premotor cortex, supplementary motor area, insular cortex, anterior cingulate cortex, fusiform gyrus, and for
for a Biotype 4 depression from pairwise fMRI functional connectivity measures between neuroanatomical brain regions including the fusiform gyros, parahippocampal cortex, ventrolateral prefrontal cortex, posterior parietal cortex, anterior cingulate cortex, middle cingulate cortex, insular cortex, temporal pole, superior temporal gyros, middle temporal gyrus, orbitofrontal cortex, ventral hippocampus, subgenual anterior cingulate cortex, ventral striatum, thalamus, primary somatosensory cortex, supplementary motor area, insular cortex, postcentral gyrus, and lingual gyros.

2. The method of claim 1, wherein:
the biotype classifier comprises a plurality of classifiers;
the plurality of classifiers comprises a first set of classifiers, each classifier configured to generate a biotype depression likelihood score for a different corresponding depression biotype;
the depression biotype is identified by selecting the depression biotype associated with the classifier that generates the highest biotype depression likelihood score based on the extracted brain region functional connectivity information; and
the extracted brain region functional connectivity information comprises functional connectivity levels between a plurality of the following patient brain regions: the dorsomedial prefrontal cortex, middle temporal gyrus, parahippocampal cortex, dorsolateral prefrontal cortex, anterior prefrontal cortex, posterior parietal cortex, anterior cingulate cortex, insular cortex, ventrolateral prefrontal cortex, precuneus, orbitofrontal cortex, ventral striatum thalamus, raphe nucleus (midbrain), primary cortex, somatosensory cortex, and visual cortex.

3. The method of claim 2, further comprising processing the extracted brain functional connectivity information by an additional classifier included in a second set of classifiers configured for determining if the patient suffers from depression, wherein the additional classifier is associated with the depression biotype identified for the patient.

4. The method of claim 1, wherein the biotype classifier comprises a linear support vector machine classifier configured to output the likelihood of an associated depression biotype.

5. The method of claim 1, further comprises classifying a depression treatment prognosis for the patient by applying a prognosis classifier to the extracted brain region functional connectivity information, wherein the prognosis classifier is configured to output a likelihood of success of an identified depression treatment for the patient.

6. The method of claim 5, wherein identifying the likelihood of success of an identified depression treatment for the patient comprises processing the extracted brain region functional connectivity information between a plurality of the following patient brain regions: the dorsomedial prefrontal cortex, anterior cingulate cortex, posterior cingulate cortex, ventromedial prefrontal cortex, ventrolateral prefrontal cortex, dorsolateral prefrontal cortex, posterior parietal cortex, orbitofrontal cortex, amygdala, ventral striatum, nucleus accumbens, globus pallidus, thalamus, primary motor cortex, primary somatosensory cortex, and visual cortex.

7. The method of claim 1 or claim 6, wherein identifying the depression biotype or the likelihood of success of the identified depression treatment for the patient comprises performing a principal component analysis of the brain region functional connectivity extracted from the fMRI signal.

8. The method of claim 6, wherein the identified depression treatment is a repetitive transcranial magnetic stimulation.

9. The method of claim 1, further comprising predicting a depression severity of the patient by processing the extracted brain region functional connectivity information with a multiple linear regression model, wherein predicting the depression severity for a patient comprises processing the extracted brain region functional connectivity information between a plurality of the following patient brain regions: the dorsomedial prefrontal cortex, posterior cingulate cortex, precuneus, posterior parietal cortex, middle temporal gyrus, parahippocampal cortex, anterior prefrontal cortex, anterior cingulate cortex, ventrolateral prefrontal cortex, insular cortex, orbitofrontal cortex, ventral hippocampus, ventral striatum, thalamus, primary motor cortex, premotor cortex, and visual cortex.

10. A system for identifying neurophysiological biotypes of depression in the brain of a patient, adapted to perform the method of any one of claims 1-9.

11. A non-transitory computer readable medium storing a computer readable instructions, which when executed by a processor cause the processor to carry out the method of any of claims 1-9.

## Patentansprüche

1. Verfahren zur Identifizierung eines neurophysiologischen Biotyps einer Depression im Gehirn eines Patienten, umfassend:
Empfangen, durch einen Prozessor, von aufgezeichneten fMRT-Daten, die die Gehirnaktivität des Patienten anzeigen;
Extrahieren, durch den Prozessor, von Information zur funktionellen Konnektivität von Hirnregionen aus dem fMRT-Signal;
Identifizieren eines Depressionsbiotyps für den Patienten durch Anwenden eines auf dem Prozessor ausgeführten Biotyp-Klassifikators auf die aus dem fMRT-Signal extrahierten Informationen zur funktionellen Konnektivität von Hirnregionen;
Ausgabe, durch den Prozessor, des identifizierten Biotyps der Depression,
wobei das Bestimmen, ob der Patient an einer Depression vom Biotyp 1, Biotyp 2, Biotyp 3 oder Biotyp 4 leidet, aus paarweisen fMRT-Messungen der funktionellen Konnektivität zwischen verschiedenen neuroanatomischen Hirnregionen abgeleitet wird,
für eine Depression vom Biotyp 1 aus paarweisen fMRT-Messungen der funktionellen Konnektivität zwischen neuroanatomischen Hirnregionen, die die hintere Region des parietalen Kortex, den Precuneus, die mittleren temporalen Gyri, den parahippocampalen Kortex, den dorsolateralen präfrontalen Kortex, den ventrolateralen präfrontalen Kortex, den insularen Kortex, den temporalen Pol beinhalten, den oberen Temporalgyrus, den ventromedialen präfrontalen Kortex, den ventralen Hippocampus, die Amygdala, den ventralen Nucleus caudatus, das ventrale Striatum, den Thalamus, den primären somatosensorischen Kortex, den primären motorischen Kortex und den visuellen Kortex umfassen,
bei einer Depression vom Biotyp 2 anhand paarweiser fMRT-Messungen der funktionellen Konnektivität zwischen neuroanatomischen Hirnregionen, die die dorsomediale präfrontale Region, die ventromediale präfrontale Region, den orbitofrontalen Kortex, den hinteren cingulären Kortex, den fusiformen Gyrus, den mittleren temporalen Gyrus, den parahippocampaler Kortex, den anterioren cingulären Kortex, den ventrolateralen präfrontalen Kortex, den dorsolateralen präfrontaler Kortex, den insulären Kortex, den posterioren parietalen Kortex, den Thalamus, den primären somatosensorischen Kortex, den primären motorischen Kortex und den visuellen Kortex umfassen,
für eine Depression vom Biotyp 3 anhand paarweiser fMRT-Messungen der funktionellen Konnektivität zwischen neuroanatomischen Hirnregionen, die die ventromediale präfrontale Region, den hinteren parietalen Kortex, den mittleren temporalen Gyrus, den ventrolateralen präfrontalen Kortex, den vorderen präfrontalen Kortex, den dorsolateralen präfrontalen Kortex, den hinteren parietalen Kortex, den Temporallappen und den oberen temporalen Gyrus, sowie den Orbitofrontalkortex, den parahippocampalen Kortex, ventralen Hippocampus, die Amygdala, den subgenualen anterioren cingulären Kortex, den ventralen Nucleus caudatus, das ventrale Striatum, den Thalamus, das prämotorische Gebiet, ein supplementäres motorisches Gebiet, den insulären Kortex, den anterioren cingulären Kortex und den Gyrus fusiformis umfassen, sowie
für eine Depression vom Biotyp 4 anhand paarweiser fMRT-Messungen der funktionellen Konnektivität zwischen neuroanatomischen Hirnregionen, die den Gyrus fusiformis, den parahippocampalen Kortex, den ventrolateralen präfrontalen Kortex, den hinteren Parietalkortex, den vorderen cingulären Kortex, den mittleren cingulären Kortex, den insularen Kortex, den Temporallappen, den oberen temporalen Gyrus, den mittleren temporalen Gyrus, den orbitofrontalen Kortex, den ventralen Hippocampus, ein subgenuales Gebiet des anterioren cingulären Kortex, das ventrale Striatum, den Thalamus, den primären somatosensorischen Kortex, den supplementären motorischen Kortex, den insulären Kortex, ein postzentrales Gebiet und den lingualen Gyrus umfassen.

2. Verfahren nach Anspruch 1, wobei:
der Biotyp-Klassifikator eine Mehrzahl von Klassifizierern umfasst;
die Mehrzahl der Klassifizierern eine erste Menge von Klassifizierern umfasst, wobei jeder Klassifizierer dazu konfiguriert ist, einen Biotyp-Depressionswahrscheinlichkeitswert für einen jeweils anderen entsprechenden Depressions-Biotyp zu erzeugen;
der Depressions-Biotyp identifiziert wird, indem der Depressions-Biotyp ausgewählt wird, der mit dem Klassifizierer assoziiert ist, der basierend auf der extrahierten Information zur funktionellen Konnektivität der Hirnregionen den höchsten Biotyp-Depressionswahrscheinlichkeitswert erzeugt; und
die extrahierte Information zur funktionellen Konnektivität der Hirnregionen Niveaus der funktionellen Konnektivität zwischen einer Mehrzahl der folgenden Hirnregionen des Patienten umfassen: dem dorsomedialen präfrontalen Kortex, dem mittleren Temporalgyrus, dem parahippocampalen Kortex, dem dorsolateralen präfrontalen Kortex, dem anterioren präfrontalen Kortex, dem posterioren parietalen Kortex, dem anterioren cingulären Kortex, dem insularen Kortex, dem ventrolateralen präfrontalen Kortex, dem Precuneus, dem orbitofrontalen Kortex, dam ventralen Striatum, dem Thalamus, dem Raphe-Kern (Mittelhirn), dem primären Kortex, dem somatosensorischen Kortex und dem visuellen Kortex.

3. Verfahren nach Anspruch 2, das ferner die Verarbeitung der extrahierten Information zur funktionellen Konnektivität des Gehirns durch einen zusätzlichen Klassifizierer umfasst, der in einem zweiten Satz von Klassifizierern enthalten ist, die dazu konfiguriert sind, zu bestimmen, ob der Patient an einer Depression leidet, wobei der zusätzliche Klassifizierer dem für den Patienten identifizierten Depressions-Biotyp assoziiert ist.

4. Verfahren nach Anspruch 1, wobei der Biotyp-Klassifikator einen linearen Support-Vektor-Maschinen-Klassifizierer umfasst, der dazu konfiguriert ist, die Wahrscheinlichkeit eines assoziierten Depressions-Biotyps auszugeben.

5. Verfahren nach Anspruch 1, das ferner die Klassifizierung einer Prognose für die Behandlung der Depression des Patienten durch Anwendung eines Prognose-Klassifizierers auf die extrahierten Informationen zur funktionellen Konnektivität der Gehirnregionen umfasst, wobei der Prognose-Klassifizierer dazu konfiguriert ist, eine Erfolgswahrscheinlichkeit einer identifizierten Behandlung der Depression für den Patienten auszugeben.

6. Verfahren nach Anspruch 5, wobei das Ermitteln der Erfolgswahrscheinlichkeit einer identifizierten Depressionsbehandlung für den Patienten die Verarbeitung der extrahierten Information zur funktionellen Konnektivität zwischen einer Mehrzahl der folgenden Hirnregionen des Patienten umfasst: dem dorsomedialen präfrontalen Kortex, dem anterioren cingulären Kortex, dem posterioren cingulären Kortex, dem ventromedialen präfrontalen Kortex, dem ventrolateralen präfrontalen Kortex, dem dorsolateralen präfrontalen Kortex, dem posterioren parietalen Kortex, dem orbitofrontalen Kortex, der Amygdala, dem ventralen Striatum, dem Nucleus accumbens, dem Globus pallidus, dem Thalamus, dem primären motorischen Kortex, dem primären somatosensorischen Kortex und dem visuellen Kortex.

7. Verfahren nach Anspruch 1 oder Anspruch 6, wobei das Identifizieren des Depressions-Biotyps oder der Erfolgswahrscheinlichkeit der identifizierten Depressionsbehandlung für den Patienten das Durchführen einer Hauptkomponentenanalyse der aus dem fMRT-Signal extrahierten funktionellen Konnektivität der Hirnregionen umfasst.

8. Verfahren nach Anspruch 6, wobei die identifizierte Depressionsbehandlung eine repetitive transkranielle Magnetstimulation ist.

9. Verfahren nach Anspruch 1, das ferner die Vorhersage des Schweregrads der Depression des Patienten durch Verarbeitung der extrahierten Information zur funktionellen Konnektivität der Hirnregionen mit einem multiplen linearen Regressionsmodell umfasst, wobei die Vorhersage des Schweregrads der Depression für einen Patienten die Verarbeitung der extrahierten Informationen zur funktionellen Konnektivität der Hirnregionen zwischen einer Mehrzahl der folgenden Hirnregionen des Patienten umfasst: dem dorsomedialen präfrontalen Kortex, dem hinteren cingulären Kortex, dem Precuneus, dem hinteren parietalen Kortex, dem mittleren Temporalgyrus, dem parahippocampalen Kortex, dem vorderen präfrontalen Kortex, dem vorderen cingulären Kortex, dem ventrolateralen präfrontalen Kortex, dem insulären Kortex, dem orbitofrontalen Kortex, dem ventralen Hippocampus, dem ventralen Striatum, dem Thalamus, dem primären motorischen Kortex, dem prämotorischen Kortex und dem visuellen Kortex.

10. System zur Identifizierung neurophysiologischer Biotypen der Depression im Gehirn eines Patienten, das dazu ausgelegt ist, das Verfahren gemäß einem der Ansprüche 1 bis 9 durchzuführen.

11. Nicht-transitorisches, computerlesbares Medium, auf dem computerlesbare Anweisungen gespeichert sind, die, wenn sie von einem Prozessor ausgeführt werden, den Prozessor veranlassen, das Verfahren gemäß einem der Ansprüche 1 bis 9 durchzuführen.

## Revendications

1. Procédé pour identifier un biotype neurophysiologique de dépression dans le cerveau d'un patient, comprenant :
la réception, par un processeur, de données IRMf enregistrées révélatrices d'une activité cérébrale du patient ;
l'extraction, par le processeur, d'informations de connectivité fonctionnelle des régions cérébrales à partir du signal IRMf ;
l'identification d'un biotype de dépression chez le patient en appliquant un classificateur de biotypes s'exécutant dans le processeur aux informations de connectivité fonctionnelle des régions cérébrales extraites du signal IRMf ;
la sortie, par le processeur, du biotype de dépression identifié,
où la détermination du fait que le patient souffre d'un biotype parmi un Biotype 1, un Biotype 2, un Biotype 3 ou un Biotype 4 de dépression est dérivée de mesures par IRMf de connectivité fonctionnelle par paires entre différentes régions neuroanatomiques du cerveau,
pour une dépression de Biotype 1, à partir de mesures par IRMf de connectivité fonctionnelle par paires entre des régions neuroanatomiques du cerveau, incluant le cortex pariétal postérieur, le précuneus, le gyrus temporal moyen, le cortex parahippocampique, le cortex préfrontal dorsolatéral, le cortex préfrontal ventrolatéral, le cortex insulaire, le pôle temporal, le gyrus temporal supérieur, le cortex préfrontal ventromédial, l'hippocampe ventral, l'amygdale, le noyau caudé ventral, le striatum ventral, le thalamus, le cortex somatosensoriel primaire, le cortex moteur primaire, le cortex visuel,
pour une dépression de Biotype 2, à partir de mesures par IRMf de connectivité fonctionnelle par paires entre des régions neuroanatomiques du cerveau, incluant le cortex préfrontal dorsomédial, le cortex préfrontal ventromédial, le cortex orbitofrontal, le cortex cingulaire postérieur, le gyrus fusiforme, le gyrus temporal moyen, le cortex parahippocampique, le cortex cingulaire antérieur, le cortex préfrontal ventrolatéral, le cortex préfrontal dorsolatéral, le cortex insulaire, le cortex pariétal postérieur, le thalamus, le cortex somatosensoriel primaire, le cortex moteur primaire et le cortex visuel,
pour une dépression de Biotype 3, à partir de mesures par IRMf de connectivité fonctionnelle par paires entre des régions neuroanatomiques du cerveau, incluant le cortex préfrontal ventromédial, le cortex pariétal postérieur, le gyrus temporal moyen, le cortex préfrontal ventrolatéral, le cortex préfrontal antérieur, le cortex préfrontal dorsolatéral, le cortex pariétal postérieur, le pôle temporal, le gyrus temporal supérieur, le cortex orbitofrontal, le cortex parahippocampique, l'hippocampe ventral, l'amygdale, le cortex cingulaire antérieur subgénual, le noyau caudé ventral, le striatum ventral, le thalamus, le cortex prémoteur, l'aire motrice supplémentaire, le cortex insulaire, le cortex cingulaire antérieur, le gyrus fusiforme, et
pour une dépression de Biotype 4, à partir de mesures par IRMf de connectivité fonctionnelle par paires entre des régions neuroanatomiques du cerveau, incluant le gyrus fusiforme, le cortex parahippocampique, le cortex préfrontal ventrolatéral, le cortex pariétal postérieur, le cortex cingulaire antérieur, le cortex cingulaire moyen, le cortex insulaire, le pôle temporal, le gyrus temporal supérieur, le gyrus temporal moyen, le cortex orbitofrontal, l'hippocampe ventral, le cortex cingulaire antérieur subgénual, le striatum ventral, le thalamus, le cortex somatosensoriel primaire, l'aire motrice supplémentaire, le cortex insulaire, le gyrus postcentral et le gyrus lingual.

2. Procédé selon la revendication 1, où :
le classificateur de biotypes comprend une pluralité de classificateurs ;
la pluralité de classificateurs comprend un premier ensemble de classificateurs, chaque classificateur étant configuré pour générer un score de probabilité de dépression liée au biotype pour un biotype de dépression correspondant différent ;
le biotype de dépression est identifié en sélectionnant le biotype de dépression associé au classificateur qui génère le score de probabilité de dépression liée au biotype le plus élevé sur la base des informations de connectivité fonctionnelle des régions cérébrales extraites ; et
les informations de connectivité fonctionnelle des régions cérébrales extraites comprennent des niveaux de connectivité fonctionnelle entre une pluralité des régions cérébrales du patient suivantes: le cortex préfrontal dorsomédial, le gyrus temporal moyen, le cortex parahippocampique, le cortex préfrontal dorsolatéral, le cortex préfrontal antérieur, le cortex pariétal postérieur, le cortex cingulaire antérieur, le cortex insulaire, le cortex préfrontal ventrolatéral, le précuneus, le cortex orbitofrontal, le striatum ventral, le thalamus, le noyau du raphé (mésencéphale), le cortex primaire, le cortex somatosensoriel et le cortex visuel.

3. Procédé selon la revendication 2, comprenant en outre le traitement des informations de connectivité fonctionnelle cérébrale extraites à l'aide d'un classificateur supplémentaire inclus dans un deuxième ensemble de classificateurs configurés pour déterminer si le patient souffre de dépression, où le classificateur supplémentaire est associé au biotype de dépression identifié pour le patient.

4. Procédé selon la revendication 1, où le classificateur de biotypes comprend un classificateur à machine à vecteurs de support linéaire configuré pour fournir la probabilité d'un biotype de dépression associé.

5. Procédé selon la revendication 1, comprenant en outre la classification d'un pronostic de traitement de la dépression pour le patient en appliquant un classificateur de pronostic aux informations de connectivité fonctionnelle des régions cérébrales extraites, où le classificateur de pronostic est configuré pour fournir une probabilité de réussite d'un traitement de la dépression identifié pour le patient.

6. Procédé selon la revendication 5, où l'identification de la probabilité de réussite d'un traitement de la dépression identifié pour le patient comprend le traitement des informations de connectivité fonctionnelle des régions cérébrales extraites entre une pluralité des régions cérébrales suivantes du patient : le cortex préfrontal dorsomédial, le cortex cingulaire antérieur, le cortex cingulaire postérieur, le cortex préfrontal ventromédial, le cortex préfrontal ventrolatéral, le cortex préfrontal dorsolatéral, le cortex pariétal postérieur, le cortex orbitofrontal, l'amygdale, le striatum ventral, le noyau accumbens, le globus pallidus, le thalamus, le cortex moteur primaire, le cortex somatosensoriel primaire et le cortex visuel.

7. Procédé selon la revendication 1 ou la revendication 6, où l'identification du biotype de dépression ou de la probabilité de réussite du traitement de la dépression identifié pour le patient comprend la réalisation d'une analyse en composantes principales de la connectivité fonctionnelle des régions cérébrales extraite du signal IRMf.

8. Procédé selon la revendication 6, où le traitement de la dépression identifié est une stimulation magnétique transcrânienne répétitive.

9. Procédé selon la revendication 1, comprenant en outre la prédiction de la gravité de la dépression du patient par le traitement des informations de connectivité fonctionnelle des régions cérébrales extraites à l'aide d'un modèle de régression linéaire multiple, où la prédiction de la gravité de la dépression d'un patient comprend le traitement des informations de connectivité fonctionnelle des régions cérébrales extraites entre une pluralité des régions cérébrales du patient suivantes : le cortex préfrontal dorsomédial, le cortex cingulaire postérieur, le précuneus, le cortex pariétal postérieur, le gyrus temporal moyen, le cortex parahippocampique, le cortex préfrontal antérieur, le cortex cingulaire antérieur, le cortex préfrontal ventrolatéral, le cortex insulaire, le cortex orbitofrontal, l'hippocampe ventral, le striatum ventral, le thalamus, le cortex moteur primaire, le cortex prémoteur et le cortex visuel.

10. Système pour identifier des biotypes neurophysiologiques de la dépression dans le cerveau d'un patient, adapté pour mettre en œuvre le procédé selon l'une quelconque des revendications 1 à 9.

11. Support non transitoire lisible par ordinateur, sur lequel sont stockées des instructions lisibles par ordinateur qui, lorsqu'elles sont exécutées par un processeur, amènent le processeur à mettre en œuvre le procédé selon l'une quelconque des revendications 1 à 9.
